# DEMANDE DE BREVET EUROPEEN

(11) **EP 3 569 206 A1**
(43) Date de publication de la demande: **20.11.2019**
(21) Numéro de dépôt: 19174164.4
(22) Date de dépôt: 13.05.2019
(51) Int. Cl.: A61F 13/08, D04B 1/26

(54) **DISPOSITIF POUR THERAPIE COMPRESSIVE D'UN MEMBRE**

(30) Priorité: 14.05.2018 BE 201805313
(71) Demandeur: Noho.Care SA, 1000 Bruxelles (BE)
(72) Inventeur: BEAUMONT, Emilie, 1080 Molenbeek (BE); BELGRADO, Jean-Paul, 1201 Bruxelles (BE); SINECHAL, Xavier, 1970 Wezembeek-Oppem (BE); MATIA, Victor, 1370 Jodoigne (BE)
(74) Mandataire: Gevers Patents

(57) **Abrégé**

L'invention concerne un dispositif (1) pour thérapie compressive d'un membre. Le dispositif comprend des parties de lacets (70) prévues pour pouvoir être tendues par les moyens de serrage (45) de façon à rapprocher des bords (51, 52) d'une première partie du dispositif surmontant une bande d'ajustement, cet ensemble ayant une surface intérieure tubulaire agencée pour épouser une forme du membre une partie de cette surface intérieure tubulaire étant de courbure différente de zéro.

## Description

### Domaine technique

La présente invention concerne un dispositif pour thérapie compressive d'un membre.

### Art antérieur

Un lymphoedème est une augmentation du volume ainsi qu'une déformation de la géométrie d'une ou plusieurs parties d'un corps. Le lymphoedème correspond typiquement à une accumulation de fluide et de macromolécules au seing de la matrice extracellulaire et/ou à reflux de liquide lymphatique dans des tissus conjonctifs d'un humain ou d'un animal. L'oedème associé est initialement essentiellement composé de fluides. Au fil du temps, un tissu fibreux et/ou graisseux laisse toutefois place à des fluides mobilisables. Ce sont généralement des membres (supérieurs ou inférieurs) qui sont atteints de lymphoedème, mais des organes génitaux ou des régions anatomiques de la ligne médiane peuvent aussi être atteints exclusivement et/ou en même temps que les membres. Un lymphoedème peut survenir dans diverses situations cliniques. Des lymphoedèmes dits « primaires » correspondent à des insuffisances lymphatiques d'ordre génétique et/ou congénital. Des lymphoedèmes dits « secondaires » correspondent à des suite iatrogènes de traitements du cancer comme la chirurgie la radiothérapie et la chimiothérapie. Ils peuvent aussi survenir suite à un des traumatismes, des infections, et/ou des expositions à des agents toxiques.

Un humain atteint d'un lymphoedème le restera généralement toute sa vie. Un traitement connu d'un lymphoedème pour un membre d'un humain consiste en une combinaison de drainage manuel, de bandage en plusieurs couches sur les tissus atteints, et du port de bas ou de manches de contention. Ce traitement est généralement au moins partiellement assisté par un professionnel de la santé.

Ce traitement présente des désavantages. Tout d'abord, il est coûteux en matériel, en termes de déplacement et d'absentéisme dans l'activité professionnelle et sociale, ainsi qu'en prestations médicales et paramédicales. Ensuite, il est peu précis et le placement du bandage, du bas ou de la manche est difficile à mettre en oeuvre, en particulier, pour des personnes âgées. Enfin, ce traitement est peu efficace sur une longue durée. En effet, la pression à l'interface entre la peau du membre et le bandage chute rapidement réduisant ainsi l'efficacité du bandage dès la deuxième heures après son placement pour devenir quasi nulle quatre heures après son placement. En effet, le gonflement de l'oedème associé couvert par le bandage diminue compte tenu d'une décompression locale faisant suite au déplacement des fluides vers une racine du membre, hors de la zone sous la compression exercée par le bandage. Une alternative à cette perte d'efficacité serait déplacer et/ou reposer le bandage régulièrement. Malheureusement, cette alternative est très chronophage, fastidieuse et source d'augmentation du risque d'erreur de compression par la grande variabilité de pose du bandage inter et intra opérateur.

### Résumé de l'invention

Un objet de l'invention est de fournir un dispositif pour thérapie compressive d'un membre qui est particulièrement efficace, particulièrement simple à mettre en place, et dont la mise en place est particulièrement précise. Ce dispositif peut notamment être utilisé pour le traitement d'un lymphoedème d'un membre et/ou d'une insuffisance veineuse du membre.

À cet effet, l'invention propose un dispositif pour thérapie compressive d'un membre comprenant :
- une première partie comprenant :
   - un premier élément allongé,
   - un deuxième élément allongé,
   - un troisième élément allongé, et
   - des moyens d'accroche ;
   le deuxième élément étant fixé, préférentiellement cousu, avec le premier élément sur une première ligne de fixation non-rectiligne et avec le troisième élément sur une deuxième ligne de fixation non-rectiligne,
   de façon à ce que la première partie ait un premier bord longitudinal et un deuxième bord longitudinal libres ;
   les moyens d'accroche étant répartis le long du premier bord et du deuxième bord ;
- une bande comprenant :
   - un quatrième élément allongé, et
   - des moyens de serrage autobloquants répartis sur le quatrième élément ;
   le quatrième élément étant prévu pour être partiellement installé entre le membre et le premier élément et partiellement installé entre le membre et le troisième élément ;
- des parties de lacets prévues pour pouvoir être tendues par les moyens de serrage et pour pouvoir coupler mécaniquement les moyens d'accroche situés le long du premier bord et les moyens d'accroche situés le long du deuxième bord,
   de façon à ce que le premier et le deuxième bords puissent être rapprochés grâce à un serrage des parties de lacets à l'aide des moyens de serrage,
   de façon à ce que la bande et la première partie forment un ensemble ayant une surface intérieure tubulaire agencée pour épouser une partie du membre,
   une partie de cette surface intérieure étant de courbure différente de zéro.

La présence de trois éléments allongés dans la première partie, fixés entre eux sur des lignes non-rectilignes permet que la forme de la première partie épouse particulièrement bien la forme du membre. En effet, utiliser moins que trois éléments, ou les fixer selon des lignes rectilignes ne permettrait pas de suivre aussi bien la configuration géométrique tridimensionnelle d'un membre, qui est typiquement allongé et de section variable.

Cette conformation du dispositif selon l'invention à la forme du membre est encore améliorée par la courbure non-nulle d'au moins une partie de la surface intérieure de l'ensemble formé par la bande et la première partie. En effet, cette courbure non-nulle permet d'éviter que des plis ne viennent perturber la répartition de la pression sur le membre. En outre, si le dispositif est prévu pour couvrir deux côtés d'une articulation, la courbure non-nulle permet de suivre la configuration du membre, même au niveau de l'angulations à l'articulation.

Le fait que la bande puisse bouger par rapport à la première partie permet que le dispositif s'adapte facilement à la morphologie du patient. Le serrage par des moyens de serrage répartis sur la bande rend aussi le dispositif selon l'invention particulièrement efficace. En effet, le fait que les moyens de serrage soient sur la bande et pas sur un des bords de la première partie permet un meilleur équilibre entre les deux bords longitudinaux de la première partie. De plus, cet agencement permet une compression graduelle et dégressive (selon un gradient de pression dit disto-proximal) d'une partie distale du membre à une partie proximale du membre car, le degré de serrage peut être adapté en fonction de la position du moyen de serrage. Cela permet d'éviter une accumulation de fluide en excès, par exemple de liquide lymphatique dans ou à proximité d'une partie distale du membre et de favoriser son évacuation vers le reste du corps.

En outre, comme la surface intérieure de l'ensemble formé par la bande et la première partie est tubulaire, elle recouvre au moins une partie du membre sur toute sa circonférence latérale. La pression s'exerce donc sur toute la circonférence du membre, ce qui permet d'améliorer l'efficacité du dispositif.

Le dispositif selon l'invention est donc particulièrement efficace car il permet une adaptation particulièrement adéquate à la géométrie du membre, à la fois au repos et en action, ce qui permet une répartition particulièrement efficace des forces de compression appliquées sur la longueur du membre ainsi que d'établir et de maintenir un gradient de pression disto- proximal.

Ainsi, le dispositif selon l'invention est adapté à la morphologie du membre, permet son maintien naturel et suit les modifications de géométrie du membre au cours de la réduction de l'oedème associé. Le design du dispositif comprend un compromis avantageux entre une mobilité fonctionnelle du membre et une rigidité nécessaire de la partie externe du dispositif. En particulier, le dispositif selon l'invention permet une certaine mobilité du membre, ce qui permet une activité physique (ou d'une activité musculaire) du patient auquel appartient ce membre. Ainsi, le patient peut poursuivre diverses activités sociales et/ou professionnelles tout en étant équipé du dispositif. Par ailleurs, et avantageusement, l'activité physique active des mécanismes cardiovasculaires qui favorisent la circulation des fluides en excès et certains mécanismes qui contribuent au retour de ces fluides dans la grande circulation.

Un avantage de l'invention est que les moyens de serrage étant répartis sur la bande, ils sont aisément visibles par un opérateur de telle façon à les rendre facilement visibles et manipulables. Ainsi, un placement ou une adaptation du serrage du dispositif selon l'invention est rendu particulièrement facile et rapide, en particulier, pour une personne âgée. En outre, le fait que les moyens de serrage soient autobloquants rend le serrage particulièrement aisé.

De préférence, ces moyens de serrage comprennent un déclencheur de déblocage, c'est-à-dire un moyen pour un utilisateur d'enclencher un déblocage. Cela peut être par exemple un bouton à presser, ou un élément à tirer qui, lorsqu'on l'actionne, relâche le blocage. Ainsi, les moyens de serrage sont facilement débloquant pour la sécurité d'un patient. Par exemple, un relâchement immédiat du serrage est possible en cas de perception inadéquate ou de malaise du patient.

Un avantage de l'invention est que le temps de placement du dispositif selon l'invention est très court. Le membre à traiter peut être introduit très facilement dans le dispositif grâce à la bande qui protège le membre d'un contact direct avec les parties de lacets et les moyens de serrage, et permet d'écarter plus facilement ses éléments pour créer un espace à l'intérieur de la surface dans lequel le membre s'introduit facilement. Ainsi, le temps nécessaire à l'enfilement du dispositif est très réduit, et le temps de réglage du dispositif se ramène à un simple serrage approprié des moyens de serrage.

Un avantage de l'invention est que le dispositif de traitement selon la présente invention est particulièrement efficace sur une longue durée. En effet, les moyens de serrage peuvent être utilisés pour tendre les différentes parties de lacets périodiquement, de façon à ce que la pression exercée par le dispositif sur le membre soit en permanence efficace, et ce même lorsque le gonflement du membre diminue par effet de compression. En particulier, il n'est pas nécessaire d'enlever et de replacer le dispositif selon l'invention toutes les deux à quatre heures pour que le traitement qu'il prodigue soit efficace. De plus, comme les moyens de serrage sont autobloquants, il est facile d'ajuster légèrement le serrage à de nombreuses reprises, sans risquer de toute desserrer. Le dispositif selon l'invention est donc non seulement plus efficace mais son utilisation permet également un gain de temps très significatif.

Un avantage de l'invention est qu'une utilisation d'un dispositif selon l'invention est meilleur marché que le matériel et les prestations médicales nécessaires à une combinaison de drainage manuel, de bandage en plusieurs couches, et/ou du port de bas ou de manches de contention. En effet, le coût du dispositif est largement amorti sur sa durée d'utilisation, sans nécessité d'achat d'un matériel médical supplémentaire. En outre, les prestations médicales et/ou interventions paramédicales sont réduites car le membre peut être traité efficacement et simplement de manière autonome par un individu au moyen du dispositif sans faire appel systématiquement à des prestataires de soins. De plus, le coût de déplacement et/ou les absences sur les lieux travails représentant une charge non négligeable sont ainsi nettement réduits.

Ainsi, le dispositif selon l'invention est de faible coût, particulièrement efficace, particulièrement rapide et simple à mettre en place, et sa mise en place est particulièrement précise. En outre, il convient au traitement des oedèmes et plus particulièrement des lymphoedèmes et/ou d'une insuffisance veineuse d'un membre humain ou/et animal, supérieur ou/et inférieur, antérieur et/ou postérieur. Il peut convenir à toute thérapie compressive d'un membre sur avis médical.

La surface intérieure de l'ensemble formé par la bande et la première partie préférentiellement issu de l'agencement avantageux des premier, deuxième, troisième et quatrième éléments. En particulier, la première partie ne définit pas complètement la surface intérieure car elle comprend les premier et deuxième bords libres agencés pour être rapprochés, préférentiellement à une distance entre 2 et 5 cm, grâce à un serrage des parties de lacets à l'aide des moyens de serrage. Vu que le quatrième élément est prévu pour être partiellement, et préférentiellement totalement, installé entre le membre et le premier élément et entre le membre et le troisième élément, il définit une partie de la surface intérieure comprenant la partie entre les premier et deuxième bords libres. En particulier, si la partie entre les premier et deuxième bords libres définit une ouverture de la première partie, le quatrième élément se glisse entre la première partie et le membre, de façon à être surmonté par cette ouverture. Lorsque le dispositif est installé sur un membre, le premier bord et le deuxième bord se font face et sont préférentiellement approximativement parallèles.

L'ouverture susmentionnée de la première partie entre les premier et deuxième bords libres est préférentiellement présente sur tout le long de la première partie et disposée de façon à ne faire face ou dos à aucune articulation principale du membre, par exemple, un coude ou un genou. L'ouverture est donc préférentiellement située latéralement par rapport à une telle articulation principale. Ainsi, non seulement, la mobilité du membre s'en trouve améliorée mais également les moyens de serrage sont disposés sur des parties latérales visibles et accessibles du membre. À titre d'exemple, lorsque le membre consiste en membre supérieure humain, l'ouverture n'est préférentiellement située ni sur l'avant, ni sur l'arrière du membre mais latéralement de façon à permettre au patient humain de voir et de manipuler les moyens de serrage sans aucune difficulté. Ainsi, un avantage de l'invention est que le patient peut conserver un contact visuel permanent avec l'ouverture et les moyens de serrage. Le patient peut également manipuler aisément et rapidement les moyens de serrage, sans force et sans se tordre sur lui-même. Ainsi, le dispositif selon l'invention est donc d'autant plus facile, précis et rapide à utiliser, en particulier pour une personne âgée à mobilité réduite.

La géométrie de la surface intérieure et l'agencement des éléments du dispositif est particulièrement adaptée à la morphologie des membres humains, c'est-à-dire à la position anatomique fonctionnelle dite « neutre ». Ceci évite de générer des tensions inadéquates au sein des articulations et des muscles afin d'assurer un confort maximal. Le dispositif s'adapte aussi aux situations anatomiques animales. À titre d'exemple pour le membre supérieur humain : l'anatomie est telle que le segment de l'avant-bras et du bras au repos marquent, de façon naturelle une disposition légèrement coudé suivant un angle intérieur d'environ 150° et présente, par rapport à son axe, une torsion au poignet d'environ 22,5°. En outre, le membre s'articule au poignet, au coude et à l'épaule. Il s'ensuit qu'une surface de courbure nulle n'épouse pas adéquatement un membre supérieur humain. Si la surface intérieure tubulaire était un cylindre de section transverse d'aire constante, elle contraindrait les mouvements du membre de telle façon qu'une très faible mobilité du membre du patient et des risques de douleurs articulaires ou musculaires en découleraient. En particulier, dans ce cas, le dispositif assemblé a préférentiellement une forme de manche de veste. Une manche de veste définit une surface de courbure non nulle, contrairement à une manche de chemise qui est conique. Un design en manche de veste pour l'assemblage de la première partie et de la bande du dispositif est donc avantageusement adapté à l'anatomie d'un membre supérieur humain. Le dispositif assemblé a de préférence une forme de surface latérale de banane. Préférentiellement, les premier, deuxième et troisième éléments sont néanmoins obtenus à partir du découpage de matériau dans un plan avant d'être fixés ensemble, préférentiellement cousus ensemble de façon à former la première partie.

Dans le cadre de ce document, la courbure d'une surface correspond à la courbure de Gauss de cette surface. Par exemple, si une feuille de papier ne peut recouvrir la surface intérieure sans pli ou déchirure, cela signifie que la surface est courbée, au moins en certains points. À titre d'exemple, un plan, un cylindre et un tronc de cône sont des surfaces dont la courbure est nulle partout. Vu que la surface intérieure tubulaire formée par la bande et la première partie comprend une partie de courbure non nulle, cette surface tubulaire n'est pas cylindrique. En particulier, la surface intérieure tubulaire ne peut ni être posée à plat, ni être dépliée à plat.

Vu que les lignes de fixation sont non-rectilignes, elles contribuent à une conception de la première partie comme étant non-planaire.

Les premier, deuxième, troisième et quatrième éléments comprennent préférentiellement des surpiqûres sur des portions destinées à être appliquées et/ou à épouser et/ou à surmonter une articulation. Une meilleure mobilité de l'articulation du membre est ainsi avantageusement induite par ces surpiqûres.

Préférentiellement, la surface intérieure tubulaire est de section transverse d'aire variable.

Préférentiellement, la première partie et la bande sont d'une épaisseur comprise entre 0,5 et 5 cm, plus préférentiellement entre 1 et 2 cm. Un avantage de l'invention est que le dispositif selon l'invention est ainsi discret, pratique et intime à porter sous un vêtement pour un humain. Le dispositif est préférentiellement recouvert d'un revêtement esthétique. Celui-ci est préférentiellement constitué de tissus ou matériel non-élastique qui garantit la répartition correcte des forces sur le membre. Les pressions sont appliquées sur la section comprimée en adéquation avec la loi de Laplace qui tient compte du rayon de courbure des différents points le long de la section sur lesquelles la force est exercée.

Dans le cadre du présent document, un lacet est susceptible de comprendre un fil et/ou une ficelle et/ou une cordelette et/ou un câble et/ou une corde et/ou une lanière et/ou un ruban et/ou une sangle. Un lacet est susceptible d'être en tout matériau.

Dans le cadre du présent document, des moyens de serrage autobloquants sont des moyens de serrage des lacets et parties de lacets dans lesquels un serrage se bloque automatiquement, empêchant ainsi un desserrage sans une action spécifique. Un moyen de serrage autobloquant peut être, par exemple, un système de serrage à cliquet.

Il est bien connu d'un homme du métier que l'adjectif « distal » fait référence à une partie d'une portion d'un corps la plus éloignée d'un organe de référence ou d'un tronc du corps (ou d'une racine d'un membre lorsque la portion du corps est un membre). Il est également bien connu d'un homme du métier que l'adjectif « proximal » fait référence à une portion d'une partie d'un corps la plus rapprochée d'un organe de référence ou d'un tronc du corps (ou d'une racine d'un membre lorsque la portion du corps est un membre). Dans le cadre du présent document, ces définitions s'appliqueront de préférence à des portions du membre, du dispositif et des éléments qui constituent le dispositif selon l'invention. En particulier, et à titre d'exemple, lorsque le membre est un membre supérieur humain, une portion proximale du membre comprend un dessus du bras et/ou un voisinage d'une épaule de l'humain, et une portion distale du membre comprend une extrémité libre de l'avant-bras et/ou un voisinage d'une main de l'humain. Vu que le dispositif et les éléments le constituant sont prévus pour être disposés sur le membre, les définitions de portions proximale et distale du membre s'étendent préférentiellement naturellement aux différentes portions du dispositif et des éléments destinés à être disposés directement sur ces portions proximales et distales du membre. En particulier, une portion distale, une portion proximale, un bord extrémal distal et un bord extrémal proximal de l'un quelconque parmi les premier, deuxième, troisième ou quatrième éléments consistent préférentiellement en une portion ou un bord de cet élément, qui est destinée à surmonter respectivement une portion distale, une portion proximale, un bord extrémal distal et un bord extrémal proximal du membre.

Les premier, deuxième, troisième et quatrième éléments sont préférentiellement allongés le long d'un axe commun. Une longueur de l'un quelconque des premier, deuxième, troisième ou quatrième éléments correspond de façon préférentielle à une distance maximale mesurée le long de l'axe séparant un point du bord extrémal distal et un point du bord extrémal proximal de l'élément. Une largeur de l'un quelconque des premier, deuxième, troisième ou quatrième éléments sur une portion est préférentiellement mesurée perpendiculairement à l'axe.

Typiquement, dans le cas où le membre est un membre supérieur humain, une main est de préférence couverte par le dispositif s'il est posé sur l'avant-bras et l'avant-bras est de préférence couvert par le dispositif s'il est placé sur une portion proximale du bras.

Dans le cadre de ce document, le terme « bras » est utilisé pour désigner une portion d'un membre supérieur humain. Le terme « membre supérieur » fait référence anatomiquement à l'avant-bras et au bras, ainsi qu'optionnellement à la main.

Selon un mode de réalisation particulier de l'invention, les moyens d'accroche comprennent des crochets fixés au premier élément et des crochets fixés au troisième élément.

Les différentes caractéristiques de l'invention, qu'elles soient prises séparément ou en combinaison d'au moins certaines d'entre elles, permettent que l'invention soit particulièrement utile dans le cadre de n'importe quelle thérapie compressive, que ce soit un traitement d'un lymphoedème, d'une insuffisance veineuse ou autre. Dans ce document, il est noté qu'un « traitement d'une insuffisance veineuse » ne correspond pas à n'importe quel traitement d'insuffisance veineuse. En particulier, la pertinence d'un traitement tel que mentionné dans ce texte et/ou d'une utilisation du dispositif selon l'invention est à évaluer de façon systématique par un praticien professionnel selon la situation clinique du patient.

Selon un mode de réalisation particulier de l'invention, les parties de lacets sont indépendantes les unes des autres, chaque partie de lacet étant prévue pour coupler mécaniquement un des moyens d'accroche situé le long du premier bord et un des moyens d'accroche situé le long du deuxième bord,
chaque moyen d'accroche étant prévu pour accrocher une seule partie de lacets.

Préférentiellement, chaque moyen de serrage est prévu pour serrer une seule partie de lacets.

Un avantage de l'invention est qu'un serrage selon ce mode de réalisation peut se faire indépendamment en différentes positions de serrage, car à chaque position de serrage correspondent un moyen d'accroche sur le premier élément propre à cette position de serrage, un moyen d'accroche sur le deuxième élément propre à cette position de serrage, une partie de lacet propre à cette position de serrage et un moyen de serrage propre à cette position de serrage. Les moyens d'accroche consistent un système mécanique de mise en tension progressive qui induit une compression du membre.

Un avantage majeur de l'invention est qu'un gradient de pression contrôlable et indépendant de la géométrie du membre inséré peut être défini très facilement sur le membre grâce au dispositif selon l'invention en induisant des serrages d'intensité décroissante des positions de serrage dans une portion distale du dispositif, jusqu'aux positions de serrage dans une portion proximale du dispositif. De même, un ajustement du serrage du dispositif selon l'invention est rendu d'autant plus aisé.

Préférentiellement, le dispositif selon l'invention comprend entre trois et dix telles positions de serrage, plus préférentiellement, entre cinq et sept telles positions de serrage. En d'autres termes, le dispositif selon l'invention comprend préférentiellement entre trois et dix moyens de serrage, entre trois et dix parties de lacets et entre trois et dix paires de moyens d'accroche, chaque paire consistant en un moyen d'accroche sur le premier élément et un moyen d'accroche sur le deuxième élément.

Les positions de serrage susmentionnées, les moyens de serrage, les parties de lacets et/ou les paires de moyens d'accroche sont essentiellement alignés le long d'un axe le long duquel les premier, deuxième, troisième et/ou quatrième éléments sont allongés.

Préférentiellement, les parties de lacets sont solidarisées à la première partie du dispositif selon l'invention de part et d'autre de son ouverture longitudinal bordée par les bords libres. Les parties de lacets sont également solidarisées avec la bande pour faciliter l'introduction du membre à l'intérieur de la surface intérieure tubulaire du dispositif. La bande permet avantageusement une bonne tenue du membre par le dispositif et une meilleure efficacité du traitement tel qu'explicité précédemment. La bande et la première partie sont préférentiellement désolidarisées.

Selon un mode de réalisation particulier de l'invention, les moyens de serrages comprennent des éléments de serrage à cliquet faisant saillie entre le premier bord et le deuxième bord lorsque le quatrième élément est partiellement installé entre le membre et le premier élément et partiellement installé entre le membre et le troisième élément.

Préférentiellement, les éléments de serrage à cliquet consistent en des systèmes BOA® comprenant des roulettes de serrage.

Un avantage de l'invention est que de tels éléments de serrage à cliquet sont connus d'un homme du métier, peu couteux et simples à utiliser.

Selon un mode de réalisation particulier de l'invention, chacun des premier, deuxième, troisième et quatrième éléments est allongé le long d'un même axe et comprend une portion proximale et une portion distale, et en ce qu'une largueur d'au moins un des premier, deuxième, troisième et quatrième éléments mesurée transversalement à l'axe est plus grande sur la portion proximale que sur la portion distale.

Un avantage de l'invention est que les dimensions des premier, deuxième, troisième et quatrième éléments sont choisies pour fournir les effets avantageux du dispositif selon l'invention en terme de compression et de géométrie de la surface intérieure tubulaire qu'ils forment.

De préférence, les largeurs des premier, deuxième, troisième et quatrième éléments mesurées transversalement à l'axe le long duquel ils sont allongés est plus grande sur les portions proximales que sur les portions distales de ces éléments.

Selon un mode de réalisation particulier de l'invention, la première partie est concave au niveau de la première ligne de fixation et convexe au niveau de la deuxième ligne de fixation.

Un avantage de l'invention est que la géométrie des lignes de fixation induit la courbure de la surface intérieure tubulaire tout en permettant une découpe et une conception particulièrement aisée de la bande et de la première partie du dispositif selon l'invention.

Selon un mode de réalisation particulier de l'invention, chacun des premier, deuxième et troisième éléments comprend une portion proximale et une portion distale ; le deuxième élément comprend un premier renfoncement au niveau de sa portion distale ;
le troisième élément comprend un deuxième renfoncement au niveau de sa portion distale ; et
les premier et deuxième renfoncements forment un trou traversant dans la première partie.

Un avantage de l'invention est que le trou traversant est agencé pour faire passer un doigt, de préférence le pouce, ou un ou des orteils d'un patient humain, à travers la première partie.

Un avantage de l'invention est que le dispositif selon ce mode de réalisation de l'invention tient compte de la géométrie complexe du membre. Il permet d'intégrer partiellement une main ou un pied d'un patient de façon à ne pas comprimer une veine et à ne pas gêner ou blesser le patient lors du port du dispositif. Un avantage de l'invention est que la mobilité du membre du patient est ainsi conservée lors du port du dispositif sur le membre.

Selon un mode de réalisation particulier du mode de réalisation particulier précédent de l'invention, la première partie comprend :
- un bord distal bordant au moins partiellement la portion distale de chacun des premier, deuxième et troisième éléments, et
- un bord proximal bordant au moins partiellement la portion proximale de chacun des premier, deuxième et troisième éléments ;
la première partie est complètement bordée par le premier bord longitudinal, le deuxième bord longitudinal, le bord distal et le bord proximal ;
le trou est situé à proximité du bord distal de la première partie ; et
la première partie comprend un troisième renfoncement au niveau de son bord proximal.

Un avantage de l'invention est que le troisième renfoncement est agencé pour que la première partie soit adaptée à la morphologie d'une épaule et/ou d'un haut de cuisse d'un patient.

Selon un mode de réalisation préféré des modes de réalisation précédents de l'invention comprenant un trou traversant dans la première partie, le dispositif comprend en outre une sangle, de préférence à tension réglable,
fixée à la première partie,
agencée pour surmonter, préférentiellement partiellement, à la fois le quatrième élément et les portions distales des premier, deuxième et troisième éléments, et agencée pour s'enrouler au moins une fois autour de la surface intérieure tubulaire formée par la bande et la première partie,
de façon à encadrer le trou, lorsque le premier et le deuxième bords sont rapprochés grâce à un serrage des parties de lacets à l'aide des moyens de serrage.

Un avantage de l'invention est que la sangle contribue en un serrage de la portion distale du dispositif, ainsi qu'en un maintien d'une main ou d'un pied d'un patient lors du port du dispositif.

Préférentiellement, la sangle se recoupe transversalement sur elle-même lors de son enroulement autour de la surface intérieure tubulaire, de façon à former une croix de maintien pour un portion distale d'un membre.

Selon un mode de réalisation particulier de l'invention, chacun des premier, deuxième et troisième éléments est constitué d'un empilement de couches de matériaux. Préférentiellement, le quatrième élément est également constitué de l'empilement de couches de matériaux.

Préférentiellement, l'empilement est homogène et isotrope. Préférentiellement, l'empilement comprend une couche de mousse qui permet une répartition avantageuse homogène d'une pression exercée par le dispositif sur le membre, quelque soit la morphologie de celui-ci.

Les couches de matériaux permettent d'induire de façon avantageuse des propriétés et avantages de chacun des matériaux au dispositif.

Préférentiellement, l'empilement de couches comprend au moins une couche de jersey, une couche de mousse et une couche de tissus non-élastique. Le jersey consiste préférentiellement en un tissu fin tricoté, comprenant de la laine et/ou du coton et/ou des fibres synthétiques.

Plus préférentiellement, l'empilement de couches consiste en une couche de jersey, une couche de mousse, une membrane de composants électroniques, une couche de mousse et une couche de tissus non-élastique, dans cet ordre, de l'intérieur vers l'extérieur de la première partie et/ou de la bande, de façon à ce que la couche de jersey soit en contact avec le membre ou un tissus recouvrant le membre.

La répartition des mousses le long du dispositif est de façon préférée homogène. Une répartition hétérogène ne se départit toutefois pas du cadre de l'invention. En particulier, des propriétés des mousses des couches de mousse sont susceptibles de différer selon leurs positions pour tenir compte des rayons de courbure spécifiques à chaque section du membre.

Selon un mode de réalisation préféré des modes de réalisation particuliers précédents de l'invention pour lesquels chacun des premier, deuxième et troisième éléments est constitué d'un empilement de couches, l'empilement comprend au moins une membrane imprimée allongée comprenant une pluralité de capteurs de pression apte à mesurer une pression comprise entre 0 et 160 mmHg.

Un avantage de l'invention est que la membrane est apte à permettre une évaluation (en temps réel et/ou en temps différé) de la pression exercée par le dispositif sur le membre.

Un avantage de l'invention est que l'intervalle compris entre 0 et 160 mmHg est précisément l'intervalle de mesure nécessaire à une mesure de pression exercée sur un membre dans le cadre d'une thérapie compressive. Plus précisément et préférentiellement, l'intervalle est compris entre 0 et 80 mmHg dans le cas où le membre est un bras humain et l'intervalle est compris entre 0 et 160 mmHg dans le cas où le membre est une jambe humaine.

Un avantage de l'invention est que le dispositif selon ce mode de réalisation permet de s'assurer que la pression exercée sur une portion distale du membre ne dépasse pas un certain seuil de compression d'environ 76 mmHg au poignet dans le cas où le membre est un membre supérieur humain. En effet, ce seuil pourrait être néfaste pour le patient dans une situation clinique considérée.

Un avantage de l'invention est que le dispositif selon ce mode de réalisation permet de contrôler qu'un gradient de pression négatif est bien appliqué sur le membre par le dispositif. Nous entendons par gradient négatif que la pression maximale doit être exercée sur la position distale du membre et la pression la moins élevée doit être exercée sur la position proximale du membre. Comme il a été expliqué précédemment, ce contrôle permet une bonne évacuation des fluides mobilisables vers les régions anatomiques situées au-delà du dispositif tout en limitant le risque d'une accumulation de ces fluides en portion distale du membre.

Préférentiellement, les capteurs de pression sont des capteurs piézo-résistifs fixés (par exemple, cousus) et disposés de façon matricielle dans la membrane. Un avantage de l'invention est que de tels capteurs sont simples à produire et à utiliser, peu couteux et permettent une mesure de différence de résistance aux intersections de câbles incorporés dans la membrane.

Préférentiellement, la membrane est apte à fournir des mesures de pression exercée par le dispositif sur le membre, une telle mesure pour une section donnée à proximité d'une position de serrage étant obtenu par calcul d'une moyenne de mesures des capteurs de pression couvrant la section donnée. Une précision de cette pression ainsi mesurée dépend d'un niveau de résolution de la mesure de pression. Un avantage de l'invention est qu'une telle membrane comprend un grand nombre de capteurs de pression, ce qui permet donc une mesure de pression précise.

Préférentiellement, la membrane est couplée électroniquement à une carte électronique et/ou une batterie d'alimentation des capteurs. Préférentiellement, la carte électronique et/ou la batterie est fixée sur la portion proximale de la première partie du dispositif, de façon à être apte à être manipulée et/ou rechargée par un opérateur et/ou un patient.

Préférentiellement, le dispositif comprend au moins un capteur de température et/ou au moins un capteur d'humidité et/ou au moins un accéléromètre.

Chacun des au moins un capteurs de température et d'humidité forment préférentiellement un seul et même capteur, et sont au nombre de deux. Préférentiellement, un premier de ces capteurs de température et d'humidité est fixé dans une portion proximale du dispositif et un deuxième de ces capteurs de température et d'humidité est fixé dans une autre portion du dispositif.

Préférentiellement, les capteurs de température et d'humidité sont aptes à mesurer une température et une humidité à l'intérieur de la surface tubulaire, dans un voisinage du membre, au sein d'une ou plusieurs régions entre le membre et le dispositif. Un avantage de l'invention est que cette mesure de température et d'humidité est un facteur intéressant dans le cadre du traitement, car les fluctuations de température et d'humidité sont susceptibles de jouer un rôle sur l'évolution de la pathologie et/ou la vitesse de réduction d'un oedème. Outre les aspects physiologiques, une température et/ou une humidité inappropriées à l'intérieur du dispositif sont un facteur de développement de bactéries et champignons, et/ou d'une absorption de l'humidité par une mousse du dispositif et/ou altération des caractéristiques techniques des senseurs piézo-résistifs.

Dans le cas où le membre est un membre supérieur humain, le dispositif comprend de façon préférée un premier capteur de température et un premier capteur d'humidité tout deux disposés sur un segment de l'avant-bras et un deuxième capteur de température et un deuxième capteur d'humidité tout deux disposés sur un segment du bras. Avantageusement, les capteurs de température permettre de détecter des signes précoces de et/ou d'anticiper une infection du type érésipèle tant sur le segment de l'avant-bras et que sur le segment de bras. Avantageusement, les capteurs d'humidité permettent de détecter une sudation trop importante qui entraînerait une réduction de l'effet thermique au sein du dispositif.

Préférentiellement, le dispositif comprend un accéléromètre disposé en portion distale du dispositif. Un tel accéléromètre contribue avantageusement à évaluer l'intensité d'utilisation du dispositif ainsi que d'évaluer l'activité du membre du patient sur le bandage mais aussi plus globalement d'évaluer l'activité physique du patient, cette dernière jouant un rôle dans l'évolution favorable d'une thérapie compressive.

Préférentiellement, l'invention comprend également un système informatique connectés aux capteurs de pression de façon à pouvoir lui envoyer des données. Le système informatique comprend un terminal mobile d'un patient et/ou un terminal d'un praticien. De préférence, les capteurs de pression sont connectés aux deux terminaux.

Dès que le dispositif est ajusté sur le membre, la connexion des capteurs avec le système informatique permet préférentiellement et avantageusement un rétrocontrôle « feedback » qualitatif et/ou quantitatif vers le patient et/ou le praticien via un affichage sur au moins un des deux terminaux de données associées à une mesure de pression des capteurs.

Un avantage de l'invention est que ce feedback permet d'estimer des valeurs de la pression sur une collection de points donnés du membre, de façon préférée les positions de serrage susmentionnées, et d'aider un patient et/ou un praticien à :
- définir une ligne de base de pression s'étendant d'une portion distale à une portion proximale du membre de telle façon que la pression diminue de la portion distale à la portion proximale ;
- ajuster les moyens de serrage de telle façon que, si une section perpendiculaire à la ligne de base de pression croise celle-ci en un point de pression, le feedback propose des actions pour que la pression sur cette section soit dans l'intervalle de plus ou moins 5% autour du point de pression. Les actions sont préférentiellement de serrer, déserrer, ou de ne pas modifier au moins un des moyens de serrage.

Préférentiellement, le terminal mobile affiche un gradient de pression exercée à l'intérieur du dispositif et peut signaler une variation significative de pression, de telle sorte qu'un patient peut instantanément réagir au moyen d'un réglage des moyens de serrage du dispositif aux différentes positions de serrage.

Préférentiellement, un praticien prestataire de soins du patient reçoit des données de pression des capteurs en temps réel (et/ou en temps différé). Ces données sont préférentiellement utilisées pour construire automatiquement un historique pour faciliter une analyse et une interprétation de l'évolution du traitement pour le praticien, ceci pour optimiser et/ou personnaliser au mieux un traitement dispensé par le dispositif selon ce mode de réalisation préféré de l'invention.

Dans le cadre de ce document, il est maintenant présenté une méthode d'ajustement sur un membre d'un dispositif selon l'une quelconque des modes de réalisation de l'invention comprenant au moins une membrane imprimée allongée comprenant une pluralité de capteurs de pression apte à mesurer une pression comprise entre 0 et 160 mmHg. Cette méthode comprend les étapes suivantes :
- fournir :
   - le dispositif ;
   - le membre ;
   - un système informatique apte à :
      •• recevoir des premières données introduites par un utilisateur, les premières données comprenant une ligne de base de pression le long du membre,
      •• recevoir des deuxièmes données de pressions mesurées en temps réel de la pluralité de capteurs de pression,
      •• établir une comparaison des premières et deuxièmes données,
      •• déduire de la comparaison une indication pour chacun des moyens de serrage, l'indication consistant en un parmi :
         ••• serrage trop fort du chacun des moyens de serrage,
         ••• serrage trop faible du chacun des moyens de serrage,
         ••• serrage correct du chacun des moyens de serrage ;
   - une interface connectée au système informatique, et apte à communiquer l'indication pour chacun des moyens de serrage à l'utilisateur ;
- placer le dispositif autour du membre ;
- serrer les moyens de serrage de façon à ce que la surface intérieure épouse la forme du membre ;
- introduire les premières données dans le système informatique ;
- recevoir les deuxièmes données au moyen du système informatique ;
- établir la comparaison des premières et deuxièmes données au moyen du système informatique ;
- déduire de la comparaison l'indication pour chacun des moyens de serrage au moyen du système informatique ;
- communiquer l'indication pour chacun des moyens de serrage à l'utilisateur au moyen de l'interface ;
- effectuer un ajustement de chacun des moyens de serrage sur base de l'indication, l'ajustement consistant en un parmi :
   ••• desserrer le chacun des moyens de serrage, si l'indication consiste en un serrage trop fort du chacun des moyens de serrage,
   ••• serrer le chacun des moyens de serrage, si l'indication consiste en un serrage trop faible du chacun des moyens de serrage,
   ••• laisser le chacun des moyens de serrage en position, si l'indication consiste en serrage correct du chacun des moyens de serrage.

Le système informatique permet de fournir un feedback au placement du dispositif. Malgré qu'une ligne de pression adaptée soit définie au placement du dispositif, un écart avec cette ligne de base de pression ne peut être exclu au placement du dispositif. Un tel écart peut avoir des conséquences néfastes pour le patient en termes d'efficacité du traitement et de la qualité et la direction de l'évacuation de fluide en excès. Une application de la méthode décrite permet un feedback après le placement du dispositif de telle façon qu'il est possible de déterminer un écart par rapport à la ligne de base de pression et de savoir sans difficulté comme serrer ou desserrer les moyens de serrage pour corriger cet écart.

Un avantage de cette méthode est qu'elle permet donc un traitement plus efficace et plus sûr de la pathologie. La méthode peut être utilisée pour ajuster la compression exercée par le dispositif sur le membre en cours de traitement. Cette application est d'autant plus justifiée que le dispositif doit être réajusté après placement compte tenu du fait que la taille de la grosseur du membre atteint d'un lymphoedème diminue par compression sur cet intervalle de temps.

En outre, les différents avantages mentionnés pour le dispositif selon l'invention s'appliquent mutatis mutandis à la méthode.

Un autre avantage de la méthode d'ajustement est qu'elle consiste entre autres en une méthode de tests de placement d'un dispositif selon l'invention ou de bandages médicaux pour des apprentis médecins. La méthode ne constitue donc pas exclusivement une méthode de pratique et/ou de traitement médicaux, mais aussi une méthode très utile dans l'éducation et la formation, ainsi que pour l'acquisition de données scientifiques faisant défaut. En particulier, dans ce cas, le membre est préférentiellement un membre artificiel d'un mannequin utilisé pour des tests d'apprentissage médicaux. La méthode est particulièrement utile pour permettre à de jeunes praticiens de s'exercer au placement d'un dispositif selon l'invention qui ne comprendraient pas de membrane de capteurs de pression. Le coût d'un dispositif selon l'invention muni d'une telle membrane et d'un système informatique tel que décrit est en effet plus élevé et des personnes défavorisées devraient se limiter à l'usage d'un dispositif dans une version la plus basique, néanmoins avantageuse au sens décrit en début de résumé. Ainsi, la méthode permettrait à des praticiens de se former adéquatement au placement d'un dispositif sans membrane de capteurs grâce à une formation au moyen d'un dispositif muni d'une membrane de capteurs permettant un feedback au placement.

De façon générale, il peut être introduit une utilisation d'un dispositif selon un mode quelconque de réalisation de l'invention pour traitement d'un lymphoedème d'un membre et/ou traitement d'une insuffisance veineuse d'un membre par thérapie compressive.

### Brève description des figures

D'autres caractéristiques et avantages de la présente invention apparaîtront à la lecture de la description détaillée qui suit pour la compréhension de laquelle on se reportera aux figures annexées parmi lesquelles :
- la figure 1 illustre une vue schématique des premier, deuxième, troisième et quatrième éléments d'un dispositif pour thérapie compressive d'un membre supérieur humain selon un mode de réalisation de l'invention ;
- la figure 2 illustre une vue schématique des premier, deuxième et troisième éléments cousus ensemble d'un dispositif pour thérapie compressive d'un membre supérieur humain selon un mode de réalisation de l'invention ;
- la figure 3 illustre une vue en coupe transversale d'un dispositif pour thérapie compressive d'un membre supérieur humain selon un mode de réalisation de l'invention ;
- la figure 4A illustre une vue de face d'un dispositif pour thérapie compressive d'un membre supérieur humain selon un mode de réalisation de l'invention ;
- la figure 4B illustre une vue arrière d'un dispositif pour thérapie compressive d'un membre supérieur humain selon un mode de réalisation de l'invention ;
- la figure 5 illustre une vue simplifiée d'un empilement de couches de matériaux constituant une première partie et/ou une bande d'un dispositif pour thérapie compressive d'un membre supérieur humain selon un mode de réalisation de l'invention.

Les dessins des figures ne sont pas à l'échelle. Généralement, des éléments semblables sont dénotés par des références semblables dans les figures. Dans le cadre du présent document, les éléments identiques ou analogues peuvent porter les mêmes références. En outre, la présence de numéros de référence aux dessins ne peut être considérée comme limitative, y compris lorsque ces numéros sont indiqués dans les revendications.

### Modes de réalisation de l'invention

La présente invention est décrite avec des réalisations particulières et des références à des figures mais l'invention n'est pas limitée par celles-ci. Les dessins ou figures décrits ne sont que schématiques et ne sont pas limitants.

En particulier, l'invention est décrite ci-dessous sous la forme d'un mode de réalisation consistant en un dispositif pour thérapie compressive d'un membre supérieur humain (ce dernier comprenant un bras et un avant-bras). Cette réalisation n'est aucunement limitative de la portée de l'invention en tant que telle. En particulier, l'invention peut être réalisée comme dispositif de thérapie compressive d'une jambe humaine ou d'un membre antérieur ou postérieur d'un animal.

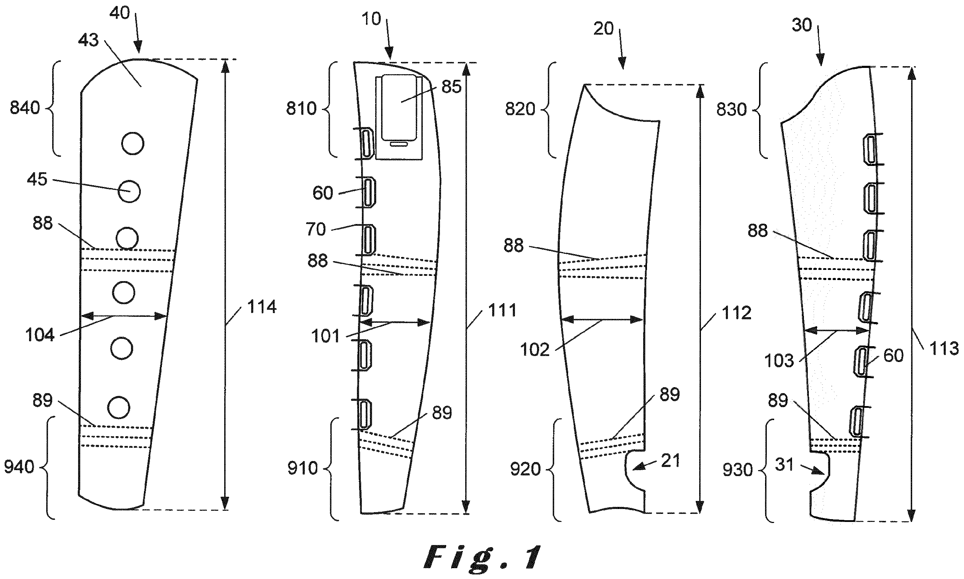

La présente invention est décrite ci-dessous sur base des représentations des figures 1 à 5 comme même mode de réalisation du dispositif.

La figure 1 une vue schématique des premier 10, deuxième 20, troisième 30 et quatrième 43 éléments d'un dispositif 1 pour thérapie compressive d'un membre supérieur humain 100 selon ce mode de réalisation. Il s'agit d'une représentation de patrons des premier 10, deuxième 20, troisième 30 et quatrième 43 éléments dans un plan. Le quatrième élément 43 consiste essentiellement en une bande 40. Les éléments 10, 20, 30 et 43 sont allongées le long d'un axe le long duquel sont mesurés leurs longueurs respectives 111, 112, 113 et 114, préférentiellement comprises entre 55 et 65 cm. Les éléments 10, 20, 30 et 43 comprennent des surpiqûres 88 transversales à l'axe sur une portion destinée à épouser et surmonter le coude, ainsi que des surpiqûres 89 transversales à l'axe sur une portion distale destinée à épouser et surmonter les articulations du poignet du membre supérieur 100. Une meilleure mobilité de l'articulation du membre supérieur 100 est ainsi induite par ces surpiqûres 88, 89 lorsque les éléments 10, 20, 30 et 43 sont assemblées pour former le dispositif 1. Le quatrième élément 43 est prévu pour être installé partiellement entre le membre supérieur 100 et les premier et troisième éléments 10 et 30, de façon à obstruer une ouverture entre ces éléments lorsque la première partie 50 est assemblée. Les éléments 10, 20, 30 et 43 comprennent chacun une portion proximale 810, 820, 830, 840 et une portion distale 910, 920, 930, 940 respectivement. Les largueurs respectives 101, 102, 103 et 104 des éléments 10, 20, 30 et 43 mesurées transversalement à l'axe sont respectivement plus grandes sur les portions proximales 810, 820, 830, 840 que sur les portions distales 910, 920, 930, 940, de façon à tenir compte de la morphologie du membre supérieur 100 étant plus large au niveau de l'épaule qu'au niveau du poignet. La largueur 104 est préférentiellement comprise entre 8 et 10 cm au niveau de la portion distale 940 et entre 15 et 16 cm au niveau de la portion proximale 840.

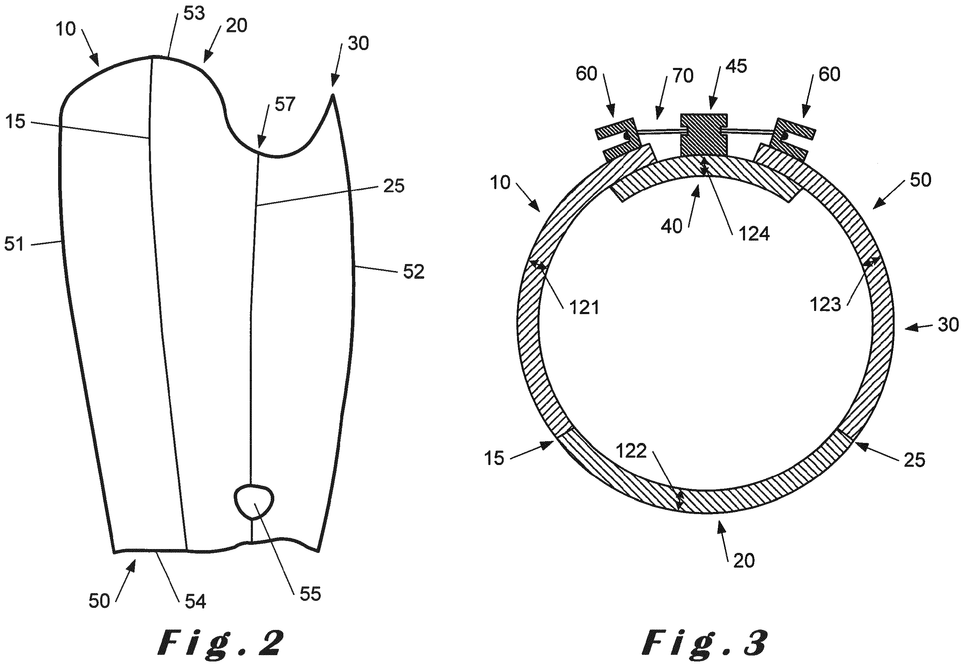

Lorsque les premier 10, deuxième 20 et troisième 30 éléments sont cousus le long des lignes de fixation 15, 25, ils forment la première partie 50 du dispositif 1. La figure 2 illustre une vue schématique de cette première partie 50 assemblée le long des lignes non-rectilignes 15, 25 ce qui induit une courbure de cette partie. La première partie 50 comprend quatre bords externes la bordant : premier 51 et un deuxième 52 bords latéraux longitudinaux et libres allongés le long de l'axe, un bord proximal 53 et un bord distal 54 qui sont prévus pour être disposés respectivement autour de l'épaule et du poignet du membre supérieur 100 lorsque le dispositif 1 est ajusté. Le bord 51 est un bord longitudinal de l'élément 10, le bord 52 est un bord longitudinal de l'élément 30, le bord 53 est un bord proximal des éléments 10, 20 et 30, et le bord 54 est un bord distal des éléments 10, 20 et 30. Le bord proximal 53 comprend un renfoncement 57 agencé pour border l'épaule du membre supérieur 100 lorsque le dispositif 1 est ajusté. La première partie 50 comprend également un trou 55 traversant formé par des renfoncements 21 et 31 respectivement dans les deuxième 20 et troisième 30 éléments. Le trou 55 est agencé pour faire passer le pouce de la main du membre supérieur 100. Cette première partie 50 intègre ainsi la main du patient et tient compte de la morphologie complexe du membre supérieur 100 décrite dans le résumé. Le trou 55 définit préférentiellement une forme elliptique ou une forme d'oeuf dont les diamètres sont compris entre 5 et 6,5 cm.

Tel qu'illustré en figure 1, le dispositif 1 comprend des moyens d'accroche 60, comprenant des paires de crochets réparties le long des premier 51 et deuxième 52 bords, un premier crochet d'une paire sur l'élément 10 faisant fasse à un deuxième crochet de la paire sur l'élément 30. La bande 40 comprend des moyens de serrage 45 autobloquants répartis sur le long d'une droite parallèle à l'axe sur le quatrième élément 43. Le dispositif 1 comprend également des parties de lacets 70 prévues pour pouvoir être tendues par les moyens de serrage 45 et pour pouvoir coupler mécaniquement les crochets situés le long du premier bord 51 et les crochets situés le long du deuxième bord 52, de façon à ce que les premier 51 et deuxième 52 bords puissent être rapprochés grâce à un serrage des parties de lacets à l'aide des moyens de serrage 45. Chaque moyen d'accroche 60 est prévu pour accrocher une seule partie de lacets 70 et chaque moyen de serrage 45 est prévu pour serrer une seule partie de lacets 70. Ainsi, l'ensemble formé par bande 40 et la première partie 50 a une surface intérieure tubulaire de courbure non nulle agencée pour épouser la forme du membre supérieur. Un diamètre intérieur de cette surface transverse à l'axe peut être ainsi réduit grâce à un serrage des moyens de serrage 45 de façon à contrôler la compression induite par le dispositif 1 sur le membre supérieur 100.

La figure 3 illustre une vue en coupe transversale du dispositif 1 assemblé au niveau d'une position de serrage telle que définie dans le résumé. En particulier, les moyens de serrages 45 comprennent des éléments de serrage à cliquet faisant saillie entre le premier bord 51 et le deuxième bord 52. La partie de lacets 70 en cette position de serrage est agencée entre les deux crochets des moyens d'accroche 60.

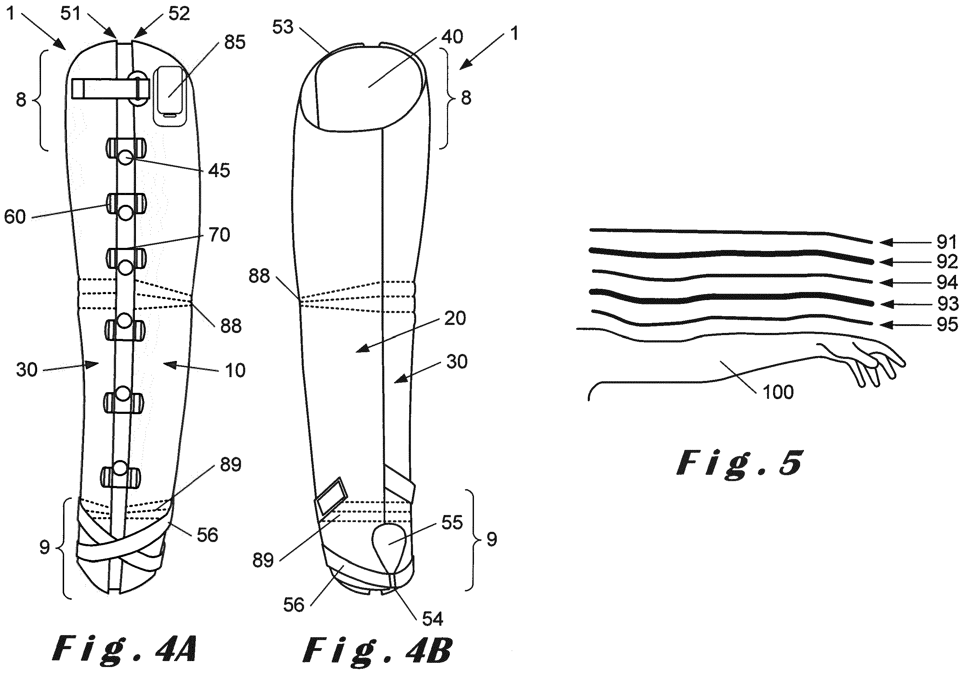

Les épaisseurs respectives 121, 122, 123 et 124 des éléments 10, 20, 30 et 43 sont sensiblement égales et comprises entre 1 et 2 cm. Elles trouvent leur origine dans un empilement de couches de matériaux 91, 92, 93, 94, 95 dont ces éléments sont constitués tel qu'illustré en figure 5. Cet empilement comprend une couche de tissus non-élastique 91 recouvrant la surface du dispositif pour des raisons esthétiques et assurant la transmission des pressions sur le membre, une couche de jersey 95 destiné à être en contact avec le membre supérieur 100 ou un tissus recouvrant le membre supérieur 100, et deux couches de mousses 92, 93 recouvrant les deux faces d'une membrane 94 de capteurs de pression dont les effets avantageux sont décrits dans le résumé.

Les figures 4A et 4B illustrent des vues d'ensemble de face et d'arrière du dispositif 1 assemblé selon l'invention, celui-ci comprenant des portions proximale 8 et distale 9 respectivement formées des portions proximales 810, 820, 830 et 840, et des portions distales 910, 920, 930 et 940. L'ouverture entre les bords 51 et 52 est vue de face en figure 4A. Un membre supérieur 100 dont la forme serait épousée par la surface tubulaire intérieure à l'ensemble par la première partie 50 et la bande 40, aurait un coude qui ne ferait ni face sur la figure 4A ou 4B. Dans les deux représentations, le coude serait orienté vers le côté droit ou gauche. Ceci permet un accès visuel et une manipulation aisée des moyens de serrage à un patient. Le dispositif comprend une sangle 56 constituant une croix de maintien pour la main agencée dans un voisinage du trou 55 tel que décrit dans le résumé. Les capteurs de la membrane 94 sont connectés à une carte électronique 85 sanglée à la portion proximale 8 et accessible par le patient.

En d'autres termes, l'invention se rapporte à un dispositif 1 pour thérapie compressive d'un membre 100. Le dispositif 1 comprend des parties de lacets 70 prévues pour pouvoir être tendues par les moyens de serrage 45 de façon à rapprocher des bords 51, 52 d'une première partie 50 du dispositif 1 surmontant une bande 40 d'ajustement, cet ensemble ayant une surface intérieure tubulaire agencée pour épouser une forme du membre 100, une partie de cette surface intérieure tubulaire étant de courbure différente de zéro.

La présente invention a été décrite en relation avec des modes de réalisations spécifiques, qui ont une valeur purement illustrative et ne doivent pas être considérés comme limitatifs. D'une manière générale, il apparaîtra évident pour un homme du métier que la présente invention n'est pas limités aux exemples illustrés et/ou décrits ci-dessus. L'invention comprend chacune des caractéristiques nouvelles ainsi que toutes leurs combinaisons.

Dans le cadre du présent document, les termes « premier », « deuxième », « troisième » et « quatrième » servent uniquement à différencier les différents éléments et n'impliquent pas d'ordre entre ces éléments. L'usage des verbes « comprendre », « inclure », « comporter », ou toute autre variante, ainsi que leurs conjugaisons, ne peut en aucune façon exclure la présence d'éléments autres que ceux mentionnés. L'usage de l'article indéfini « un », « une », ou de l'article défini « le », « la » ou « l' », pour introduire un élément n'exclut pas la présence d'une pluralité de ces éléments.

## Revendications

1. Dispositif (1) pour thérapie compressive d'un membre (100) comprenant :
• une première partie (50) comprenant un premier élément (10) allongé, un deuxième élément (20) allongé, un troisième élément (30) allongé, et des moyens d'accroche (60) ; le deuxième élément (20) étant fixé avec le premier élément (10) sur une première ligne de fixation (15) non-rectiligne et avec le troisième élément (30) sur une deuxième ligne de fixation (25) non-rectiligne, de façon à ce que la première partie (50) ait un premier bord (51) longitudinal et un deuxième bord (52) longitudinal libres ; les moyens d'accroche (60) étant répartis le long du premier bord (51) et du deuxième bord (52) ;
• une bande (40) comprenant un quatrième élément (43) allongé et des moyens de serrage (45) autobloquants répartis sur le quatrième élément (43) ; le quatrième élément (43) étant prévu pour être partiellement installé entre le membre (100) et le premier élément (10) et partiellement installé entre le membre (100) et le troisième élément (30) ;
• des parties de lacets (70) prévues pour pouvoir être tendues par les moyens de serrage (45) et pour pouvoir coupler mécaniquement les moyens d'accroche (60) situés le long du premier bord (51) et les moyens d'accroche (60) situés le long du deuxième bord (52), de façon à ce que le premier (51) et le deuxième (52) bords puissent être rapprochés grâce à un serrage des parties de lacets à l'aide des moyens de serrage (45), de façon à ce que la bande (40) et la première partie (50) forment un ensemble ayant une surface intérieure tubulaire agencée pour épouser une partie du membre (100), une partie de cette surface intérieure étant de courbure différente de zéro.

2. Dispositif (1) selon la revendication 1, dans lequel les parties de lacets (70) sont indépendantes les unes des autres, chaque partie de lacet (70) étant prévue pour coupler mécaniquement un des moyens d'accroche (60) situé le long du premier bord (51) et un des moyens d'accroche (60) situé le long du deuxième bord (52), chaque moyen d'accroche (60) étant prévu pour accrocher une seule partie de lacets (70).

3. Dispositif (1) selon la revendication précédente, dans lequel chaque moyen de serrage (45) est prévu pour serrer une seule partie de lacets (70).

4. Dispositif (1) selon l'une quelconque des revendications précédentes, dans lequel les moyens de serrages (45) comprennent des éléments de serrage à cliquet faisant saillie entre le premier bord (51) et le deuxième bord (52) lorsque le quatrième élément (43) est partiellement installé entre le membre (100) et le premier élément (10) et partiellement installé entre le membre (100) et le troisième élément (30).

5. Dispositif (1) selon l'une quelconque des revendications précédentes, dans lequel les moyens d'accroche (60) comprennent des crochets fixés au premier élément (10) et des crochets fixés au troisième élément (30).

6. Dispositif (1) selon l'une quelconque des revendications précédentes, **caractérisé en ce que** chacun des premier (10), deuxième (20), troisième (30) et quatrième (43) éléments est allongé le long d'un même axe et comprend une portion proximale (810, 820, 830, 840) et une portion distale (910, 920, 930, 940),
et **en ce qu'**une largueur (101, 102, 103, 104) d'au moins un des premier (10), deuxième (20), troisième (30) et quatrième (43) éléments mesurée transversalement à l'axe est plus grande sur la portion proximale (810, 820, 830, 840) que sur la portion distale (910, 920, 930, 940).

7. Dispositif (1) selon l'une quelconque des revendications précédentes, **caractérisé en ce que** la première partie (50) est concave au niveau de la première ligne de fixation (15) et convexe au niveau de la deuxième ligne de fixation (25).

8. Dispositif (1) selon l'une quelconque des revendications précédentes, **caractérisé en ce que** chacun des premier (10), deuxième (20) et troisième (30) éléments comprend une portion proximale (810, 820, 830) et une portion distale (910, 920, 930) ;
**en ce que** le deuxième élément (20) comprend un premier renfoncement (21) au niveau de sa portion distale (920) ;
**en ce que** le troisième élément (30) comprend un deuxième renfoncement (31) au niveau de sa portion distale (930) ;
et **en ce que** les premier et deuxième renfoncements (21, 31) forment un trou (55) traversant dans la première partie (50).

9. Dispositif (1) selon la revendication précédente, **caractérisé en ce que** la première partie (50) comprend un bord distal (54) bordant au moins partiellement la portion distale (910, 920, 930) de chacun des premier (10), deuxième (20) et troisième (30) éléments, et un bord proximal (53) bordant au moins partiellement la portion proximale (810, 820, 830) de chacun des premier (10), deuxième (20) et troisième (30) éléments ;
**en ce que** la première partie (50) est complètement bordée par le premier bord (51) longitudinal, le deuxième bord (52) longitudinal, le bord distal (54) et le bord proximal (53) ;
**en ce que** le trou (55) est situé à proximité du bord distal (54) de la première partie (50) ;
et **en ce que** la première partie (50) comprend un troisième renfoncement (57) au niveau de son bord proximal.

10. Dispositif (1) selon l'une quelconque des revendications 8 ou 9, **caractérisé en ce qu'**il comprend en outre une sangle (56) fixée à la première partie (50), agencée pour surmonter partiellement à la fois le quatrième élément (43) et les portions distales des premier (10), deuxième (20) et troisième (30) éléments, et agencée pour s'enrouler au moins une fois autour de la surface intérieure de l'ensemble formé par la bande (40) et la première partie (50), de façon à encadrer le trou (55), lorsque le premier (51) et le deuxième (52) bords sont rapprochés grâce à un serrage des parties de lacets (70) à l'aide des moyens de serrage (45).

11. Dispositif (1) selon l'une quelconque des revendications précédentes, **caractérisé en ce que** chacun des premier (10), deuxième (20) et troisième (30) éléments est constitué d'un empilement de couches de matériaux (91, 92, 93, 94, 95).

12. Dispositif (1) selon la revendication précédente, **caractérisé en ce que** l'empilement comprend au moins une couche de jersey (95), une couche de mousse (92, 93) et une couche de tissus non-élastique (91).

13. Dispositif (1) selon l'une quelconque des revendications 11 ou 12, **caractérisé en ce que** l'empilement comprend en outre au moins une membrane (94) imprimée allongée comprenant une pluralité de capteurs de pression apte à mesurer une pression comprise entre 0 et 160 mmHg.

14. Dispositif (1) selon la revendication précédente, **caractérisé en ce qu'**il comprend en outre au moins un capteur de température et/ou au moins un capteur d'humidité et/ou au moins un accéléromètre.
